(19) Europäisches Patentamt

European Patent Office

Office européen des brevets

(11) Publication number: **0 556 685 A2**

(12) **EUROPEAN PATENT APPLICATION**

(21) Application number: 93101950.9

(22) Date of filing: **08.02.93**

(51) Int. Cl.⁵: **G01N 33/569, C12Q 1/32**

(30) Priority: **20.02.92 US 839142**

(43) Date of publication of application:
**25.08.93 Bulletin 93/34**

(84) Designated Contracting States:
**AT BE CH DE DK ES FR GB GR IT LI LU NL PT SE**

(71) Applicant: **MILES INC.**
**One Mellon Center 500 Grant Str.**
**Pittsburgh, PA 15219-2502(US)**

(72) Inventor: **Miller, Carol A.**
**51101 Winding Water Lane N.**
**Elkhart, Indiana 46514(JP)**

(74) Representative: **Dänner, Klaus, Dr. et al**
**Bayer AG, Konzernverwaltung RP, Patente Konzern**
**D-51368 Leverkusen (DE)**

(54) **Diagnosis of trichomonas vaginitis by detecting formate in vaginal fluid.**

(57) A method for the presumptive detection of Trichomonas in vaginal fluid, thereby providing an aid to the diagnosis of Trichomonas-caused vaginitis. A sample of vaginal fluid is combined with a test composition comprising an indicator system that provides a chromogenic response in the presence of formate; the detection of an elevated level of formate indicating the presumptive presence of Trichomonas. The test composition preferably comprises the enzyme formate dehydrogenase, NAD, an electron transfer agent, and a chromogenic indicator.

## BACKGROUND OF THE INVENTION

The present invention relates to the diagnosis of Trichomonas vaginitis. In particular, the invention concerns the presumptive detection of Trichomonas in vaginal fluid by means of a chemical assay.

Trichomonas vaginalis is a sexually transmitted protozoan parasite which causes vaginitis in women and, to a lesser extent, urethritis in men. This microorganism is not part of the endogenous flora of the genitourinary tract and, therefore, is considered an invasive pathogen. Whereas a common symptom associated with trichomonas-caused vaginitis is a profuse frothy discharge, a definitive diagnosis cannot be based upon signs and symptoms alone (Fouts, A.C. and Draus, S.J., Trichomonas vaginalis: Re-evaluation of Its Clinical Presentation and Laboratory Diagnosis, J. Infect. Dis. 141:137-143, 1980). Because of the low cost of and ease with which microscopy can be done on fresh vaginal specimens during the patient visit, direct wet mount microscopy has been widely used to detect the motile trichomonads. However, the diagnostic accuracy of direct microscopy is poor, being in the range of 56-70% (Fouts, A.C. and S.J. Kraus, Trichomonas vaginalis: Re-evaluation of Its Clinical Presentations and Laboratory Diagnosis, J. Infect. Dis. 141:137-143, 1980; Schmid, G.P. and S.A. Larsen, Finding the Elusive T. vaginalis, Diag. Med. Nov.-Dec.:38-40, 1984; Spence, M.R., et al. The Clinical and Laboratory Diagnosis of Trichomonas vaginalis Infection, Sex. Transm. Dis. 7:168-171, 1980), since accuracy depends upon visualization of motile trichomonads, i.e., non-motile trichomonads are not discernible by wet mount microscopy. Growth of these protozoans in culture media improves the accuracy of detection considerably (Schmid, G.P., et al., Evaluation of Six Media for the Growth of Trichomonas vaginalis from Vaginal Secretions, J. Clin. Microbiol. 27:1230-1233, 1989), but the lapse of time (72 hours) for culture results makes this test of low diagnostic utility. Equally as sensitive, but requiring a more sophisticated fluorescence microscope, is acridine orange staining of vaginal specimens (Hipp, S.S., et al., Screening for Trichomonas vaginalis Infection by use of Acridine Orange Fluorescent Microscopy, Sex. Transm. Dis. 6:235-238, 1979); this test has gained some favor for use with mail-in slides when fluorescence microscopes are not available to the clinician or a more sensitive assay is desired.

Immunological approaches to the diagnosis of trichomonas-caused vaginitis have been reported increasingly, but the extent and nature of the antigenic heterogeneity of Trichomonas vaginalis isolates has caused slow progress. Whereas one high molecular weight cell surface glycoprotein has been demonstrated (Alderete, J.F. et al., Monoclonal Antibody to a Major Surface Glycoprotein Immunogen Differentiates Isolates and Subpopulations of Trichomonas vaginalis, Inf. Imm. 52:70-75, 1986) to segregate isolates according to surface markers, it has little diagnostic significance. Consequently, the majority of reported immunoassay tests are based upon use of Trichomonas whole cells as immunogen to develop antibodies for direct detection of either the whole Trichomonas cell in a vaginal specimen (Smith, R.F., Detection of Trichomonas vaginalis in Vaginal Specimens by Direct Immunoflourescence Assay, J. Clin. Microbiol. 24:1107-1108, 1986) or cellular antigens of the trichomonads present in the vaginal fluid (Watt, R.J., Rapid Assay for Immunological Detection of Trichomonas vaginalis, J. Clin. Microbiol. 24:551-555, 1986; Sibau, L., et al., Enzyme-linked Immunosorbent Assay for the Diagnosis of Trichomoniasis in Women, Sex. Transm. Dis. 14:216-220, 1987; Carney, J.A., et al., New Rapid Latex Agglutination Test for Diagnosing Trichomonas vaginalis Infection, J. Clin. Path. 41:806-808, 1988).

Nitrosamines have been identified in vaginal secretions of women having trichomoniasis (Allsobrook, A.J.R., et al., Nitrosamines in the Human Vaginal Vault, IARC Scientific Pub. 9:197, 1975) with implications of cancer causation, but no reference has been made to their diagnostic value for detection of trichomonas-caused vaginitis. A cell-detaching factor (Garber, G.E., Isolation of a Cell-Detaching Factor of Trichomonas vaginalis, J. Clin. Microbiol. 27:1548-1553, 1989) was shown to be produced by this protozoan and its importance as a diagnostic tool for vaginitis was noted. Isoenzyme patterns have been investigated (Proctor, E. M. Isoenzyme Patterns of Isolates of Trichomonas vaginalis from Vancouver, Sex. Transm. Dis. 15:181-185, 1988) and found to have little relevance as a vaginitis diagnostic. While propionic acid and isovaleric acid are observed in culture fluids as end metabolic products of Trichomonas vaginalis growth, these metabolites have been discussed (Ishiguro, T. Studies on Propionic Acid, Butyric Acid, and Valeric Acid in Trichomonas vaginalis Culture Fluid, Acta Obst. Gynecol. Jpn. 36:363-368, 1984) as contributors to the promotion of cervical or vaginal cancer but not as potential diagnostic indicators of vaginitis. Similarly, in this same publication, formic acid is stated to be produced in large quantities by Trichomonads grown on culture media and is thought to give rise to strong inflammatory responses observed in the vaginal mucosa. However, the possible diagnostic significance of this metabolite was not suggested or discussed, nor the detection thereof in a biological fluid.

2

## SUMMARY OF THE INVENTION

It has now been found that Trichomonas-caused vaginitis can be presumptively identified by the simple chromogenic detection of formate in vaginal fluid. The method involves the combination of a sample of vaginal fluid with a test composition comprising an indicator system that produces a chromogenic response to the presence of formate. Such test composition preferably comprises the enzyme formate dehydrogenase, the enzyme cofactor nicotinamide dinucleotide (NAD), an electron transfer agent, and a chromogenic indicator that produces a color change in the presence of reduced NAD (NADH) and the electron transfer agent. The presence of increased levels of formate in vaginal fluid correlates with Trichomonas vaginitis in patients. In contrast with urine and serum samples which are normally sterile, vaginal fluid samples will be expected to contain products and debris from a variety of flora; however, it has been found that significant metabolism of Trichomonas-originating formate does not occur.

The ability to rapidly and simply detect formate in vaginal fluid specimens significantly aids in the diagnosis of Trichomonas vaginitis. Such a test method provides a number of important advantages over the prior diagnostic methods. For example, the detection of the simple formate metabolite does not require that intact and viable cells be isolated from the patient as is required by classical methods based on direct microscopy and culture. In addition, typical microscopy methods require significant training of the reader and the ability to obtain a sample that contains an observable number of organisms, e.g., at least $10^5$ per milliliter. Moreover, it has been found that the multitude of extraneous substances present in vaginal fluid specimens produce no significant interference with the formate dehydrogenase enzymatic reaction. Accordingly, a chromogenic assay is possible which can be very simply performed by relatively untrained individuals in a physician's office or clinic.

## BRIEF DESCRIPTION OF THE DRAWINGS

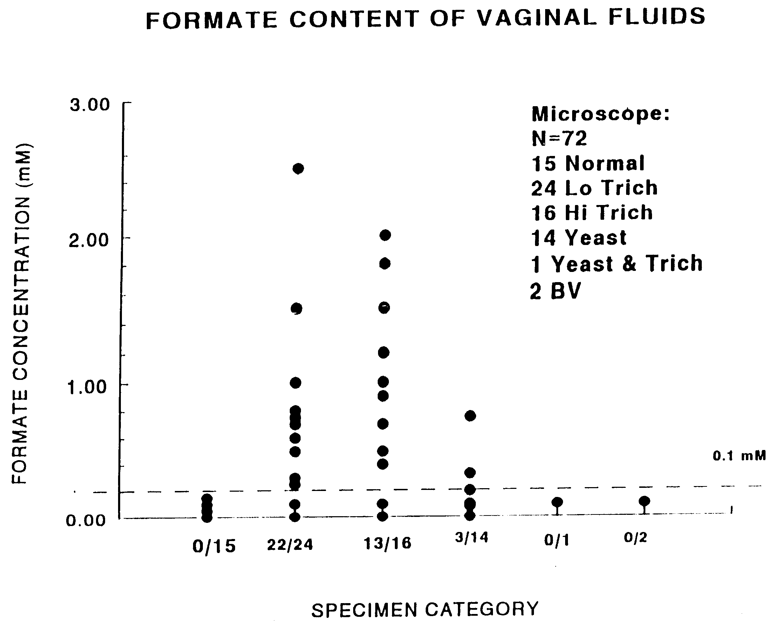

The drawing is a graph showing the results of a clinical study correlating levels of vaginal fluid formate with the clinical status of the patient.

## DESCRIPTION OF THE PREFERRED EMBODIMENTS

The vaginal sample can be obtained from a patient by a variety of known methods and can be brought into contact with the test composition with or without processing. Typically, the sample will be obtained by swab which can then be used to directly contact the sample with the test composition, e.g., by immersing the swab in a liquid containing one or more of the test reagents or by rolling or otherwise contacting the swab with the surface of a test device, e.g., test strip, in or on which one or more of the test reagents are disposed. Another approach involves the immersion of the swab into a processing or extraction liquid, e.g., comprising buffers, preservatives, or the like, and removal of an aliquot of supernatant directly from the resulting liquid mixture, or after filtering, centrifuging, or the like to remove particulate debris, or simply after allowing debris to settle by gravity. The aliquot of supernatant is then contacted with the test composition as above. In general, the method by which the sample is obtained and processed, if at all, is not critical and can be selected according to the particular needs or desires of the user.

In general, any test composition that will provide a chromogenic response in the presence of formate can be used as the test composition in the present method. Normally, the test reaction will be enzymatic and involve an enzyme such as formate dehydrogenase (FDH). Such enzyme is characterized by catalysis of the oxidation of formate in the presence of NAD with the resulting reduction of NAD to NADH. Suitable enzymes are available from a variety of sources, and most commonly will be microbial in origin. Microbial-derived FDH can be obtained by isolation and purification of the enzyme protein from suitable bacteria, yeast, fungi, or the like, e.g., Pseudomonas oxalaticus, Vibrio succinogenes , or from a strain containing a recombinant expression vector into which the gene encoding FDH, or an active fragment thereof, has been inserted by means of genetic engineering.

When using an FDH assay, the produced NADH can be detected or measured, qualitatively or quantitatively, either directly by its native color (absorption at about 340 nm), or by further reaction of NADH to produce a chromogenic species. Commonly, NADH can be determined by reaction with a variety of chromogenic indicators in the presence of a conventional electron transfer agent. Indicators and electron transfer agents that are suitable for this purpose are well known in the art. Typically, the indicator is a tetrazolium salt such as 2-(4-iodophenyl)-3-(4-nitrophenyl)-5-phenyl-tetrazolium chloride (INT), 3-(4,5-dimethylthiazol-2-yl)-2,5-diphenyltetrazoliumchloride (MTT), 2,2',5,5'-tetraphenyl-3,3'-(3,3'-dimethoxy-4,4'-diphenylene) ditetrazolium chloride (NBT), and derivatives and modifications thereof. Representative of

EP 0 556 685 A2

3

electron transfer agents are diaphorase (dihydrolipoamide reductase EC 1.6.4.3), 5-methylphenazinium methyl sulfate (PMS) and derivatives and modifications thereof, and the like. The choices of indicator and electron transfer agent are not critical and can be left to the desires and needs of the ordinary skilled worker. Further background regarding such detection systems can be found in the literature, e.g., Babson et al, Clin. Chim. Acta 12:210-215 (1965), Gay et al Clin. Chem. 14:740-753 (1968), and Gapps II et al, Clin. Chem. 12:406-413 (1966).

A particularly useful form of test composition is that of a test strip, i.e., a test device comprising a carrier substrate incorporated with the test composition. Suitable carrier substrates, as well as methods and structures for incorporating the test composition components with such carrier substrates are within the ordinary skill in the art. Examples of carrier substrates are absorbent or bibulous matrices such as filter paper, fleeces, porous and absorbent paper, cloth, glass fiber filters, polymeric membranes and films, and the like. Incorporation methods include impregnation of a formed carrier matrix with a solution, suspension, or other liquid form of the test composition, in one or more steps, followed by drying of the matrix; formation of a matrix in the presence of one or more of the components of the test composition, e.g., by casting or layering solutions of film or membrane forming formulations, or by weaving filaments containing reagents.

The present invention will now be illustrated, but is not intended to be limited by, the following examples.

## EXAMPLES

### Assay Reagents

The test system was based on the oxidation of formate by formate oxidoreductase (EC 1.2.1.2, Pseudomonas oxalaticus) and NAD with the concomitant production of NADH. Whereas the production of NADH from NAD can be measured by an absorbance change at 340 nm, in this example the enzymic reaction was coupled to a diaphorase-containing formazan producing chromogenic system to enable visual observation of color formation.

The composition of the wet chemistry reagent system was as follows:

| Stock Reagent Concentration | Test Volume (µL) |
|---|---|
| 0.2 M Sodium Phosphate Buffer (pH 7.0) | 625 |
| DMSO:$H_2O$ (1:1; v/v) | 270 |
| 50 mM NAD in $H_2O$ | 25 |
| 4 mM (INT) in $H_2O$ | 20 |
| 1.44 U/µL Diaphorase in 100 mM Tris (pH 8.5) | 10 |
| (The above reagents are mixed into a cocktail and the cocktail held at 35°C until used) | |
| 10 U/µL Formate Dehydrogenase (FDH) in 0.2 M Sodium Phosphate Buffer (pH 7.0), containing 2 mM NAD | 25 |

Assay Method

To conduct an individual test, the following procedure was used:

1. 925 $\mu$L of the FDH-less cocktail was added to a cuvette and background absorbance monitored at 500 nm and the cuvette blanked.

2. 50 $\mu$L of vaginal fluid specimen was added to the same cuvette, contents mixed and the rate of absorbance change monitored at 500 nm.

3. After one minute, this reaction mixture becomes the background, i.e., the cuvette was blanked.

4. 25 $\mu$L of FDH was added to the same cuvette and the rate of absorbance change at 500 nm again measured. Readings were completed with 30-60 seconds.

The amount of formate present in a specimen is determined by reference to a formate standard curve or directly from rate measurements.

The test has also been conducted by first combining the FDH with the reagent cocktail, blanking the cuvette followed by direct addition of vaginal fluid. This approach does not enable compensation for background interference, but comparable readings are obtained by both approaches. Specimen clarification is only necessary when specimens contain particulate matter and color formation is measured instrumentally, since color formation can be distinguished from particles when the chromogenic response is determined visually--this was the case in tests conducted, i.e., each cuvette was also observed visually for color response.

(NOTE: For those specimens having particulate matter, a brief period of centrifugation was used to clarify the specimen. In these instances, 50 $\mu$L of the supernatant was tested for formate concentration.)

Results

Vaginal fluid specimens were obtained from 72 different persons attending a clinic for routine gynecological examination or for diagnosis of suspected gynecologic infection. Prior to testing, specimens were processed as follows: vaginal mucosal exudate was obtained on a cotton or dacron swab, the swab immersed in 0.5 mL - 1.0 mL of saline (0.9% NaCl in water) and agitated to detach the exudate and to suspend/solubilize the material in the saline. Following microscopic observation of an aliquot of the fluid (fluid gas placed on a microscope slide and viewed under a microscope), the fluid was frozen at -20°C. Prior to testing, the specimen was thawed at room temperature.

The results of studies conducted on the 72 specimens are shown in the drawing which identifies six different categories of specimens. All specimens excepting those in the "Normal" category are defined by the original microscopic diagnosis; as well, patients in these categories exhibited some symptom of vaginal infection which caused them to seek a gynecologic diagnosis. Patients in the "Normal" category attended the clinic for routine gynecologic examination and exhibited no symptoms of vaginal infection or microscopic evidence of infection. All specimens in the "Normal" category contained less than 0.1 mM formate; this was also the lower limit of detection of this test format. A large percentage (85.4%) of the 41 specimens said to contain motile trichomonads contained formate in concentrations above that of normal specimens while formate was detected in only 3/14 yeast-containing specimens which had no observable motile trichomonads. Only one specimen said to contain motile trichomonads had no measurable formate.

The present invention has been particularly described and exemplified above. Clearly, many other variations and modifications of the invention can be made without departing from the spirit and scope hereof.

**Claims**

1. A method for aiding the diagnosis of Trichomonas-caused vaginitis, characterized in that:
   (a) a sample of vaginal fluid is combined with a test composition comprising an indicator system that produces a chromogenic response in the presence of formate, and
   (b) said chromogenic response is observed for elevated levels of formate in said sample.

2. The method of claim 1 wherein said sample is obtained by a swab.

3. The method of claim 2 wherein said swab is immersed in an extraction liquid and an aliquot of the supernatant is combined with said test composition.

4. The method of any of claims 1 to 3 wherein said test composition comprises formate dehydrogenase, NAD, an electron transfer agent, and a chromogenic indicator compound that produces a color change in the presence of reduced NAD and said electron transfer agent.

5. The method of claim 4 wherein said formate dehydrogenase is isolated from a bacterial source, preferably Pseudomonas oxalaticus.

6. A test composition for aiding the diagnosis of Trichomonas-caused vaginitis, characterized in that it comprises formate dehydrogenase, NAD, an electron transfer agent, and a chromogenic indicator compound that produces a color change in the presence of reduced NAD and said electron transfer agent.

7. The test composition of claim 7 wherein said formate dehydrogenase is isolated from a bacterial source, preferably Pseudomonas oxalaticus.

8. A test device for aiding the diagnosis of Trichomonas-caused vaginitis, characterized in that it comprises:
   (a) the test composition of claim 6 or 7, and
   (b) a carrier substrate incorporated therewith.

9. The test device of claim 8 wherein said carrier substrate is an absorbent matrix.

10. Use of the test composition of any of Claims 6 and 7 or af the device of any of Claims 8 and 9 in the diagnosis of Tricomonas-caused vaginitis.

# FORMATE CONTENT OF VAGINAL FLUIDS

**Microscope:**
**N=72**
**15 Normal**
**24 Lo Trich**
**16 Hi Trich**
**14 Yeast**
**1 Yeast & Trich**
**2 BV**

FORMATE CONCENTRATION (mM)

3.00

2.00

1.00

0.00

0.1 mM

0/15    22/24    13/16    3/14    0/1    0/2

SPECIMEN CATEGORY

EP 0 556 685 A2